# EUROPÄISCHE PATENTSCHRIFT

(11) **EP 1 912 934 B1**
(45) Veröffentlichungstag und Bekanntmachung des Hinweises auf die Patenterteilung: **26.02.2014**
(21) Anmeldenummer: 06792617.0
(22) Anmeldetag: 31.07.2006
(51) Int. Cl.: C07C 263/20, C07C 263/10, C07C 265/14

(54) **Verfahren zur Herstellung von Diisocyanaten**
Method for producing diisocyanates
Procédé de production de diisocyanates

(30) Priorität: 04.08.2005 DE 102005037328
(43) Veröffentlichungstag der Anmeldung: 23.04.2008
(60) Teilanmeldung: 11163223.8
(73) Patentinhaber: BASF SE, 67056 Ludwigshafen (DE)
(72) Erfinder: WÖLFERT, Andreas, 74906 Bad Rappenau (DE); KNÖSCHE, Carsten, 67150 Niederkirchen (DE); HEILIG, Manfred, 67346 Speyer (DE); STROEFER, Eckhard, 68163 Mannheim (DE)
(86) Internationale Anmeldenummer: PCT/EP2006/064850
(87) Internationale Veröffentlichungsnummer: WO 2007/014936

(56) Entgegenhaltungen:
- EP-A1- 0 593 334
- EP-A1- 0 699 657
- EP-A2- 0 150 435
- WO-A-2004/058689
- WO-A-2005/115974
- WO-A-2005/123665
- DE-A1- 10 261 191

## Beschreibung

Die vorliegenden Erfindung betrifft ein Verfahren zur Herstellung von Diisocyanaten aus Diaminen und Phosgen in der Gasphase.

EP 570799, Beispiel 1 beschreibt die Aufarbeitung eines durch Gasphasenphosgenierung erhaltenen Reaktionsgemisches mittels eines mit Wasser berieselten Waschturms zur Abtrennung von Phösgen und Chlorwasserstoff.

Durch eine derartige Aufarbeitung werden überschüssiges Phosgen sowie Chlorwasserstoffgas vernichtet und können nicht mehr nutzbringend in die Reaktion eingesetzt werden.

EP 593334 B1 und EP 699657 B1 offenbaren die Möglichkeit Phosgen oder Chlorwasserstoffgas zu verwerten oder zu zerstören, ohne jedoch auf die spezielle Problematik von rückgeführtem Phosgen einzugehen.

EP 749 958 B1 [0018] und EP 1078918 B1 [0018] erwähnen die Möglichkeit, nach Beendigung einer Gasphasenphosgenierung von Triaminen überschüssiges Phosgen rückzugewinnen und dabei wiedergewonnenes Chlorwasserstoffgas wieder in die Phosgensynthese einzusetzen.

WO 2005/123665 A1 beschreibt ein Verfahren zur Herstellung von Isocyanaten durch Umsetzung von Aminen mit Phosgen in der Gasphase. Beschrieben wird eine Quenchzone, in der das Reaktionsgemisch, das im wesentlichen aus den Isocyanaten. Phosgen und Chlorwasserstoff besteht, intensiv mit einer Flüssigkeit vermischt wird, so daß das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben. Diese Gasphase kann weiter, beispielsweise destillativ, aufgereinigt werden und das so erhaltene Phosgen wieder der Reaktion zugeführt werden.

Auch hier werden keine Angaben gemacht, die das rückgeführte Phosgen näher beschreiben.

US 4,581,174 beschreibt die kontinuierliche Herstellung von organischen Mono- und/oder Polyisocyanaten durch Phosgenierung des primären Amines in einem Mischkreis unter teilweiser Rückführung der isocyanathaltigen Reaktionsmixtur, wobei der HCl-Anteil in der rückgeführten Mischung kleiner als 0,5 % ist. Auch hier gilt, dass die kontinuierliche Rückführung des Isocyanates in die Reaktionszone mit freiem Amin die Harnstoffbildung fördert. Der ausfallende Harnstoff gefährdet den stabilen Betrieb des Verfahrens.

GB 737 442 beschreibt die Rückgewinnung von Phosgen aus der Isocyanatsynthese. Das rückgewonnene Phosgen hat einen HCl-Gehalt von 0,5 bis 0.7 %.

DE 10261191 A1 und WO 2004/58689 beschreiben Phosgenierungen, bei denen der HCl-Gehalt im phosgenhaltigen Eduktstrom weniger als 0,4 bzw. mehr als 0,8 Gew% enthält.

WO 2005/115974 beschreibt ein Verfahren zur Herstellung von Isocyanaten durch Umsetzung von Aminen mit Phosgen, wobei der phosgenhaltige Eduktstrom im wesentlichen frei von Isocyanaten ist und einen Massengehalt an Chlorwasserstoff von weniger als 0,4 Massen-% aufweist.
Diese Schriften differenzieren in der Problematik nicht zwischen Gas- und Flüssigphasenphosgenierung und beziehen sich bevorzugt nur auf Flüssigphasenphosgenierung.

Da nämlich rückgeführtes Phosgen immer einen gewissen Anteil an Chlorwasserstoff enthält, werden durch die Rückführung solchen Phosgens in die Phosgenierung von Aminen aus Amin und Chlorwasserstoff die entsprechenden Aminhydrochloride gebildet. Diese Aminhydrochloride sind im Reaktionsgemisch einer Phosgenierung, die in flüssiger Phase durchgeführt wird, oft schwer löslich, dies bringt jedoch in der Regel keine gravierenden Nachteile, beispielsweise infolge Belagbildung, mit sich, da durch die Bewegung der flüssigen Phase etwaige Belege schnell wieder entfernt werden. Im Gegenteil ist es sogar eine verbreitete Möglichkeit, die Reaktivität des eingesetzten Amins gegenüber Phosgen abzuschwächen, indem man das Amin als Hydrochlorid in die Phosgenierung einsetzt.

Bei einer Phosgenierung in der Gasphase ist die Problematik jedoch eine völlig andere: Da das gasförmige Reaktionsgemisch keine ausreichende abrasive Wirkung aufweist, können ausgefällte Belegungen, beispielsweise von Aminhydrochloriden, nicht ohne weiteres wieder entfernt werden, so daß Abscheidungen den Wärmeübergang oder die Durchströmung der betroffenen Bauteile unerwünscht verändern.

Aufgabe der Erfindung war es, ein Verfahren bereit zu stellen, das in der Umsetzung von Diaminen mit Phosgen zu den korrespondierenden Diisocyanaten und Chlorwasserstoff (HCl) in der Gasphase eine Reaktionsführung zuläßt, in der während der Reaktion sich absetzende Feststoffe vermieden werden können so daß eine hohe Raum-Zeit-Ausbeute erhalten werden kann.

Die Aufgabe wurde gelöst durch ein Verfahren zur Herstellung von Diisocyanaten durch Umsetzung der korrespondierenden Diamine mit Phosgen im stöchiometrischen Überschuß von Phosgen in mindestens einer Reaktionszone,
wobei man die Reaktionsbedingungen so wählt, daß zumindest die Reaktionskomponenten Diamin, Diisocyanat und Phosgen bei diesen Bedingungen gasförmig sind, und wobei man der Reaktionszone mindestens einen diaminhaltigen und mindestens einen phosgenhaltigen Gasstrom zuführt,
wobei man aus dem erhaltenen, im wesentlichen gasförmigen Reaktionsgemisch überschüssiges Phosgen und entstandenes Chlorwasserstoffgas (HCl) abtrennt,
man das abgetrennte, überschüssige Phosgen zumindest teilweise wieder in die Reaktion zurückführt,
und wobei man aus dem zurückgeführten Phosgenstrom Chlorwasserstoff so abtrennt, daß der Massenbruch von Chlorwasserstoff im phosgenhaltigen Strom vor der Vermischung mit dem aminhaltigen Strom mindestens 0,0001 Gew% und weniger als 15 Gew.-% beträgt.

Bei der Gasphasenphosgenierung ist es erfindungsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamoychloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Amino-gruppen und Chlorwasserstoff (HCl) bilden, da die resultierenden Aminhydrochloride leicht ausfallen und nur schwer wieder verdampfbar sind.

Durch das erfindungsgemäße Verfahren wird erreicht, daß die Bildung von Aminhydrochloriden entscheidend verringert werden kann, so daß die Gefahr von Belagsbildungen im Reaktor reduziert wird.

Gemäß dem erfindungsgemäßen Verfahren erfolgt die Umsetzung von Phosgen mit Diamin in der Gasphase. Unter Umsetzung in der Gasphase ist zu verstehen, dass die Eduktströme im gasförmigen Zustand miteinander reagieren.

Diisocyanate, die mit dem erfindungsgemäßen Verfahren hergestellt werden können, können aromatische, cycloaliphatische oder aliphatische sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate steht im Rahmen dieser Anmeldung kurz für cycloaliphatische und/oder aliphatische Isocyanate.

Beispiele für aromatische Diisocyanate sind sind bevorzugt solche mit 6 - 20 C-Atomen, beispielsweise monomeres Methylen-di(phenylisocyanat (MDI), 2,4- und/oder 2,6-Toluylendiisocyanat (TDI) und Naphtyldiisocyanat (NDI).

Diisocyanate sind bevorzugt (cyclo)aliphatische Diisocyanate besonders bevorzugt (cyclo)aliphatische Diisocyanate mit 4 bis 20 C-Atomen.

Beispiele für übliche Diisocyanate sind aliphatische Diisocyanate wie Tetramethylen-diisocyanat, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4), 8 (bzw. 9)-Bis(isocyanatomethyl)-tricyclo[5.2.10^{2.6}]decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(iso-cyanatomethyl)cyclohexan, 2,4-, oder 2,6-Diisocyanato-1-methylcyclohexan.

Bevorzugt sind 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanatocyclohexyl)-methan.

Für das erfindungsgemäße Verfahren Können solche Amine zur Reaktion zu den korrespondierenden Diisocyanaten eingesetzt werden, die bevorzugt ohne Zersetzung in die Gasphase überführt werden können. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (HDA), als 2,4- oder 2,6-Isomer oder als Gemisch davon, Diaminobenzol, R,S-1-Phenylethylamin, 1-Methyl-3-phenylpropylamin, 2,6-Xylidin, 3,3'-Diaminodiphenylsulfon, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-HDA.

Die Edukte oder auch nur eines von ihnen können zusammen mit einem Inertmedium in den Reaktionsraum eindosiert werden.

Dem erfindungsgemäßen Verfahren kann ein zusätzliches Inertmedium beigesetzt werden. Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Dichlorbenzol, Xylol, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin und/oder zu Phosgen mehr als 0,0001 und bis zu 30, bevorzugt mehr als 0,01 und bis zu 15, besonders bevorzugt mehr als 0,1 und bis zu 5 beträgt.

Bevorzugt wird das Inertmedium zusammen mit dem Diamin in den Reaktionsraum eingeführt.

Die Zuführung von Phosgen über den phosgenhaltigen Strom zum Reaktionsraum kann auch so erfolgen, dass man statt eines einzelnen phosgenhaltigen Stroms mehrere phosgenhaltige Teilströme zuführt. In einem solchen Fall werden die phosgenhaltigen Teilströme zu einem gesamten phosgenhaltigen Gesamtstrom addiert, und der Massenbruch des Chlorwasserstoffs im phosgenhaltigen Gesamtstrom ergibt sich aus den Massenbrüchen des Chlorwasserstoffs in den einzelnen phosgenhaltigen Teilströmen unter Annahme der Molekülerhaltung ohne Reaktion. In diesem Fall wird der so berechnete Wert des Chlorwasserstoff-Massenbruchs im gedanklichen Phosgen-Gesamtstrom verwendet.

Derartige Teilströme können in folgender Weise dosiert werden:
- Man kann verschiedene phosgenhaltige Teilströme, beispielsweise rückgeführtes Phosgen und Frisch-Phosgen, vor der Einspeisung zu einem phosgenhaltigen Gesamtstrom vereinigen und in den Reaktionsraum einspeisen.
- Man kann mehrere Teilströme, wobei es sich jeweils um rückgeführtes Phosgen, Frisch-Phosgen oder Gemische daraus handeln kann, an der gleichen Stelle in den Reaktionsraum einspeisen, beispielsweise über mehrere Düsen, die parallel um eine Zentraldüse angeordnet sind, wie es beispielsweise in EP 1449826 A1 beschrieben ist, oder durch mehrfache Eindüsung in einen Ringraum zur Vermischung, bevor dieser Strom mit einem über eine Zentraldüse dosierten aminhaltigen Strom vermischt wird.
- Man kann mehrere Teilströme, wobei es sich jeweils um rückgeführtes Phosgen, Frisch-Phosgen oder Gemische daraus handeln kann, an verschiedenen Stellen des Reaktionsraums eindosieren, so daß Phosgen im Verlauf der Reaktion nachdosiert wird.

Mit dem Begriff "Frisch-Phosgen" ist damit ein phosgenhaltiger Strom bezeichnet, der nicht aus einem Phosgenierungsverfahren zurückgeführt worden ist, sondern der nach der Synthese des Phosgen, zumeist aus Chlor und Kohlenmonoxid, keine Reaktionstufe mit einer Phosgenumsetzung durchlaufen hat, in der mehr als 5 % Umsatz des in der Phosgensynthese hergestellten Phosgen erfolgt.

Werden dem Reaktionsraum ein oder mehrere zusätzliche, gasförmige phosgenfreie oder aminfreie Inertströme zugeführt, dann werden diese in der Ausübung des erfindungsgemäßen Verfahrens wie ein Teilstrom des Phosgen-haltigen Gesamtstromes in der Berechnung des Phosgen-haltigen Gesamtstroms berücksichtigt.

Zur Durchführung des erfindungsgemäßen Verfahrens kann es vorteilhaft sein, die Ströme der Reaktanten vor dem Vermischen vorzuwärmen, üblicherweise auf Temperaturen von mindestens 200 °C, bevorzugt mindestens 260 °C und besonders bevorzugt mindestens 300 °C.

In der Aminvorlage wird das Amin bevorzugt zusammen mit einem Inertmedium als Trägergas, wie beispielsweise Stickstoff, in die Gasphase überführt und in die Mischeinheit eingespeist. Das Amin kann aber auch direkt ohne Verwendung eines Inertmediums verdampft werden. Ebenfalls wird Phosgen aus der Phosgenvorlage , gegebenenfalls zusammen mit einem Inertmedium, in die Gasphase übergeführt und in die Mischeinheit geleitet.

Im erfindungsgemäßen Verfahren erfolgt die Vermischung der Reaktanden in einer Mischeinrichtung, die sich durch eine hohe Scherung des durch die Mischeinrichtung geführten Reaktionsstromes auszeichnet. Bevorzugt werden als Mischeinrichtung eine statische Mischeinrichtung oder eine Mischdüse verwendet, die dem Reaktor vorangestellt ist. Besonders bevorzugt wird eine Mischdüse verwendet.

Die Art der Mischung spielt erfindungsgemäß keine Rolle und kann auf beliebige Art und Weise erfolgen, beispielsweise wie beschrieben in EP-B1 699657. EP-A2 1319655, Sp. 1, Z. 54 bis Sp. 2, Z. 24 und Sp. 4, Z. 16 - 40, EP-A1 1275640, Sp. 3, Z. 27 - Sp. 4, Z. 5, EP-A2 1362847, Sp. 2, Z. 19 - Sp. 3, Z. 51 und Sp. 4, Z. 40 - Sp. 5, Z. 12, auf die jeweils im Umfang dieser Offenbarung ausdrücklich Bezug genommen sei.

Erfindungsgemäßen wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Nach dem Vermischen in der Mischeinheit wird das gasförmige Gemisch aus Phosgen, Amin und gegebenenfalls Inertmedium in den Reaktor geführt, wobei der Reaktor den Reaktionsraum enthält.

Die Umsetzung von Phosgen mit Amin erfolgt in einem Reaktionsraum, der im allgemeinen in einem Reaktor angeordnet ist, d.h. unter Reaktionsraum wird der Raum verstanden, in dem ein für die Ausbeute des Verfahrens relevanter Teil der Umsetzung der Edukte und Zwischenprodukte und/oder der Produkte erfolgt, in dem beispielsweise mindestens 0,5 mol% des eingesetzten Amins verbraucht und/oder des korrespondierenden Isocyanats gebildet wird, bevorzugt mindestens 1 mol%, besonders bevorzugt mindestens 3 mol%, ganz besonders bevorzugt mindestens 5 mol%, insbesondere mindestens 7 mol% und speziell mindestens 10 mol% des eingesetzten Amins verbraucht und/oder des korrespondierenden Isocyanats gebildet wird.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten moderaten Drücken standhalten. Geeignete Materialien für den Kontakt mit dem Reaktionsgemisch sind z.B. Metalle, wie Stahl, Tantal, Silber oder Kupfer, Glas, Keramik, Emaille oder homogene oder heterogene Gemische daraus. Bevorzugt werden Stahlreaktoren verwendet. Die Wände des Reaktors können glatt oder profiliert sein. Als Profile eignen sich beispielsweise Ritzen oder Wellen.

Die Reaktionszone ist die Summe aller Reaktionsräume, in der das Diamin im wesentlichen vollständig in das Diisocyanat umgewandelt wird, d.h. bevorzugt mindestens 95 mol% der eingesetzten Aminogruppen, besonders bevorzugt mindestens 97 mol%, ganz besonders bevorzugt mindestens 98 mol%, insbesondere mindestens 99 mol%, speziell mindestens 99,5 mol% und sogar mindestens 99,8 mol%. Die Reaktion kann in einer oder mehrerer Reaktionszonen durchgeführt werden, von denen jeder einzelnen mindestens ein diaminhaltiger und mindestens ein phosgenhaltiger Gasstrom zugeführt wird.

Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Bevorzugt verwendet werden Rohrreaktoren.

Ebenfalls ist es möglich im wesentlichen quaderförmige Reaktionsräume, bevorzugt Plattenreaktoren bzw, Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Technisch sinnvoll haben sich Reaktionsräume mit einem Verhältnis von Breite zu Höhe bis maximal 5000 : 1, bevorzugt 1000 : 1 erwiesen.

Die Umsetzung von Phosgen mit Amin im Reaktionsraum erfolgt bei Absolutdrücken von mehr als 0,1 bar bis weniger als 20 bar, bevorzugt zwischen 0,5 bar und 10 bar, besonders bevorzugt zwischen 0,7 bar und 5 bar, insbesondere von 0,8 bis 3 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

In einer bevorzugten Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion währenddes Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Druckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 570 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,02 Sekunden bis weniger als 1,5 Sekunden. Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchlaufen die gasförmigen Reaktionspartner den Reaktionsraum mit einer Strömungsgeschwindigkeit von 3 bis 400 Meter/Sekunde, bevorzugt von 10 bis 250 Meter/Sekunde. Durch die turbulente Strömung werden eine enge Verweilzeit mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen. Bevorzugt findet die Umsetzung adiabat statt.

Das erfindungsgemäße Verfahren wird bevorzugt einstufig durchgeführt. Darunter ist zu verstehen, dass die Vermischung und Umsetzung der Edukte in einem Schritt und in einem Temperaturbereich, bevorzugt in dem vorstehend genannten Temperaturbereich, erfolgt. Ferner wird das erfindungsgemäße Verfahren bevorzugt kontinuierlich durchgeführt.

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt. Bevorzugt wird das Isocyanat verwendet.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während über-schüssiges Phosgen, Chlorwasserstoff und gegebenenfalls das Inertmedium die Aufarbeitungsvorrichtung gasförmig durchlaufen. Als inertes Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Bevorzugt wird dabei die Temperatur des inerten Lösungsmittels oberhalb der Schmelztemperatur des zum Amin gehörigen Carbamylchlorids gehalten. Besonders bevorzugt wird dabei die Temperatur des inerten Lösungsmittels oberhalb der Lösungstemperatur des zum Amin gehörigen Carbamylchlorids im gewählten Quenchmedium gehalten.

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Ein geeigneter Quench ist beispielsweise bekannt aus EP-A1 1403248, Sp. 2, Z. 39 - Sp. 3, Z. 18, auf die im Umfang dieser Offenbarung ausdrücklich Bezug genommen sei.

In dieser Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 140 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat. Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der unveröffentlichten deutschen Anmeldung mit dem Aktenzeichen 10 2004 030164.6 und dem Anmeldedatum 22.06.2004 beschrieben.

Die Flüssigkeitströpfchen werden dazu mittels Ein- oder Zweistoffzerstäuberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (otho, para), Trichlorbenzol, Xylol, Hexan, Diethylisophthalat (DEIP), Tetrahydrofuran (THF), Dimethylformamid (DMF) und deren Gemische, bevorzugt Monochlorbenzol.

In einer besonderen Ausführungsform des erfindungsgemäßen Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie HCl und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.
In der anschließenden optionalen Reinigungsstufe wird das Isocyanat, bevorzugt durch Destillation, vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A1 10260092.

Aus dem Quench und/oder der Reinigungsstufe werden Stoffströme, die im wesentlichen aus Phosgen und/oder Chlorwasserstoffgas bestehen, erhalten. Aus zumindest einem Teil dieser Phosgen und/oder Chlorwasserstoffgas enthaltenden Stoffströme wird erfindungsgemäß Chlorwasserstoff vom Phosgen abgetrennt, so daß der Massenbruch von Chlorwasserstoff im phosgenhaltigen Strom nach gegebenenfalls durchgeführter Vermischung mit Frisch-Phosgen vor der Vermischung mit dem aminhaltigen Strom weniger als 15 Gew.-%, bevorzugt weniger als 10, besonders bevorzugt weniger als 5 Gew% beträgt.

Der phosgenhaltige Gasstrom weist einen Massengehalt an Chlorwasserstoff von mindestens 0,0001 Gew% auf, besonders bevorzugt von mindestens 0,01 Gew%, ganz besonders bevorzugt von mindestens 0,1 Gew% und insbesondere von mindestens 0,25 Gew%.

Die Trennung des Chlorwasserstoff und/oder Phosgen und/oder Lösungsmittel enthaltenden Gemisches erfolgt bevorzugt über Destillation und/oder eine Wäsche. Bevorzugt wird die Trennung in einer Kombination von einer Destillation und einer Wäsche durchgeführt.

Bevorzugte Waschmedien sind die oben als Quenchmedien aufgeführten Lösungsmittel. Besonders bevorzugt werden als Wasch- und als Quenchmedium die gleichen Lösungsmittel eingesetzt.

Bei einer kombinierten Wäsche und Destillation wird Phosgen aus dem chlorwasserstoffhaltigen Strom durch Wäsche mit einem Waschmedium, bevorzugt Toluol, Chlorbenzol oder Dichlorbenzol, ausgewaschen. Besonders bevorzugt Chlorbenzol. Dabei fällt ein mit Phosgen und Chlorwasserstoff beladesnes Waschmedium an. Die Abtrennung von Phosgen und HCl aus diesem beladenen Waschmedium nach der Wäsche erfolgt bevorzugt destillativ. Dabei erfolgt erfindungsgemäß die Abtrennung so, dass ein Phosgenstrom anfällt, der gegebenenfalls nach Vermischung mit Frischphosgen einen Gehalt von kleiner 15 Gew% HCl enthält.

Die Wäsche und die Destillation wird bei Drücken von 1 bis 10 bar absolut betrieben, bevorzugt von 1 bis 5 bar absolut.

Der Chlorwasserstoff-/Phosgentrennung kann eine Adsorbtionseineinheit, bevorzugt ein Aktivkohlefilter, im aus der Trennung abgeführten Chlorwasserstoffsstrom nachgeschalten werden, in dem Spuren des Waschmediums aus dem anfallenden Chlorwasserstoff entfernt werden.

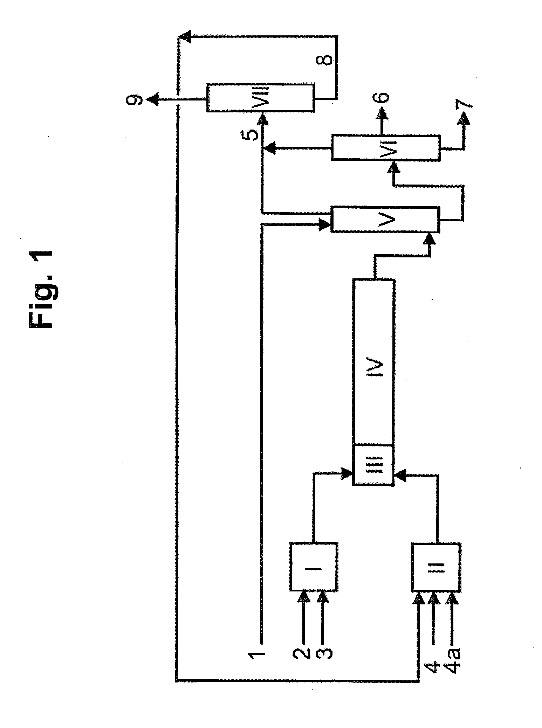

Eine bevorzugte Ausführungsform des erfindungsgemäßen Verfahrens ist in Figur 1 schematisch veranschaulicht.

In Figur 1 bedeutet:
- I: Amin-Vorlage
- II: Phosgenvorlage
- III: Mischeinheit
- IV: Reaktionsraum
- V: Aufarbeitungsstufe (Quench)
- VI: Reinigungsstufe
- VII: Phosgen-Chlorwasserstoff-Trennung
- 1: Zufuhr Lösungsmittel
- 2: Zufuhr Amin
- 3: Zufuhr Inertmedium
- 4: Zufuhr Phosgen
- 4a: Zufuhr Inertmedium
- 5: Austrag Chlorwasserstoff, Phosgen und/oder Inertmedium/Lösungsmittel
- 6: Austrag Inertmedium/Lösungsmitttel (optional)
- 7: Austrag Isocyanat
- 8: Rückführung des Phosgens
- 9: Austrag Chlorwasserstoff

In der Amin-Vorlage wird das Amin, gegebenenfalls zusammen mit einem Inertmedium als Trägergas, wie beispielsweise Stickstoff, in die Gasphase überführt und in die Mischeinheit III eingespeist. Ebenfalls wird Phosgen aus der Phosgenvorlage II in die Mischeinheit III geführt. Nach dem Vermischen in der Mischeinheit, die beispielsweise aus einer Düse oder einem statischen Mischer bestehen kann, wird das gasförmige Gemisch aus Phosgen, Amin und gegebenenfalls Inertmedium in die Reaktionszone IV überführt. Wie in Figur 1 veranschaulicht muss es sich bei der Mischeinheit nicht um eine eigenständige Reaktionsstufe handeln, vielmehr kann es vorteilhaft sein, die Mischeinheit in die Reaktionszone zu integrieren.

Nachdem das Reaktionsgemisch in der Reaktionszone umgesetzt wurde, gelangt es in die Aufarbeitungsstufe. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsstufe gasförmig durchlaufen. Als inertes Lösungsmittel sind bevorzugt aromatische Kohlenwasserstoffe, die gegebenenfalls mit Halogenatomen substituiert sind, geeignet, wie beispielsweise Chlorbenzol oder Dichlorbenzol, und Toluol. Besonders bevorzugt wird dabei die Temperatur des inerten Lösungsmittels oberhalb der Lösungstemperatur des zum Amin korrespondierenden Carbamoylchlorids im gewählten Quenchmedium gehalten.

In der anschließenden Reinigungsstufe wird das Isocyanat, bevorzugt durch Destillation, vom Lösungsmittel abgetrennt. Ebenfalls kann hier noch die Abtrennung von restlichen Verunreinigungen, beispielsweise Chlorwasserstoff, Inertmedium und/oder Phosgen erfolgen.

Erfindungsgemäß wird mindestens ein phosgenhaltiger Teilstrom 5, der aus der Reinigungsstufe und/oder der Aufarbeitungsstufe stammt, der Phosgen enthält, von darin enthaltenem Chlorwasserstoff zumindest teilweise befreit und in die Reaktion zurückgeführt. Dazu wird er mit dem Frischphosgenstrom 4 bzw. mit dem Phosgenstrom zur Mischeinrichtung III vereinigt.

Bevorzugt werden Phosgen-Rückführstrom 8 und Frischphosgenstrom 4 in einem solchen Verhältnis miteinander vermischt, daß die in der Umsetzung angestrebte Stöchiometrie erreicht wird.

In einer weiteren bevorzugten Ausführung wird dabei mindestens soviel HCl aus dem Teilstrom 5 entfernt, dass das oben angegebene Kriterium erfüllt ist.

Die Entfernung der HCl kann destillativ und/oder über eine Wäsche erfolgen. Erfolgt die Trennung über eine Wäsche und wird gleichzeitig im Verfahren ein Lösungsmittel zu auswaschen des Isocyanates am Ende der Reaktionsstrecke verwendet, dann wird als Waschmedium zur Trennung des HCl/Phosgen-Gemisches das Lösungsmittel zum Auswaschen des Isocyanates bevorzugt eingesetzt.

### Beispiele

### Beispiel 1 (erfindungsgemäß)

In einem Laborrohrreaktor mit einem Durchmesser von 12 mm und einer Länge von 1 m wird ein 1,6-Diaminoohexan (HDA)-haltiger Strom mit einem Massenstrom von 5,4 kg/hr, der mit Monochlorbenzol (MCB) vorvermischt ist, und ein phosgenhaltiger Strom mit einem Massenstrom von 16,7 kg/hr, der mit Chlorwasserstoff (HCl) vorvermischt ist, eingeleitet. Für beide Eduktströme wird eine Eingangstemperatur von 410°C eingestellt. Der Rohrreaktor wird über eine Begleitheizung so temperiert, dass ein adiabater Betrieb erfolgt.

Der HDA-haltige Strom hat einen HDA-Massengehalt von 32,5 % HDA und einen Monochlorbenzol-Massengehalt von 67,5 % MCB.

Der phosgenhaltige Strom wird so eingestellt, dass sich ein Massengehalt von 10% HCl im Gemisch HCl/Phosgen ergibt. Der HDA-haltige Strom wird über ein Einleitrohr in das Reaktionsrohr eingebracht. Ein Druck von 1,3 bar absolut wird durch eine Druckhaltung am Ausgang des Rohrreaktors eingestellt.

Der Versuch kann über 3 Tage ohne merklichen Druckanstieg in den Zuleitungen durchgeführt werden.

### Beispiel 2 (Vergleichsbeispiel, nicht erfindungsgemäß)

In einem Laborrohrreaktor mit einem Durchmesser von 12 mm und einer Länge von 1 m wird ein HDA-haltiger Strom mit einem Massenstrom von 5,4 Kg/hr, der mit Monochlorbenzol vorvermischt ist und ein phosgenhaltiger Strom mit einem Massenstrom von 18,75 kg/hr, der mit Chlorwasserstoff vorvermischt ist, eingeleitet. Für beide Eduktströme wird eine Eingangstemperatur von 410°C eingestellt. Der Rohrreaktor wird über eine Begleitheizung so temperiert, dass ein adiabater Betrieb erfolgt.

Der HDA-haltige Strom hat einen HDA-Massengehalt von 32,5 % HDA und einen Monochlorbenzol-Massengehalt von 67,5 % MCB.

Der phosgenhaltige Strom wird so eingestellt, dass sich ein Massengehalt von 20% HCl im Gemisch HCl/Phosgen ergibt.

Der HDA-Haltige Strom wird über ein Einleitrohr in das Reaktionsrohr eingebracht. Ein Druck von 1,3 bar absolut wird durch eine Druckhaltung am Ausgang des Rohrreaktors eingestellt

Es kommt zu Feststoffbildung im Rohr in der Umgebung der Einleitung des HDA, die über einen Druckanstieg im Vordruck in den Zuleitungen zum Reaktor gemessen wurde. Der Versuch wird nach einer Stunde abgebrochen.

## Patentansprüche

1. Verfahren zur Herstellung von Diisocyanaten durch Umsetzung der korrespondierenden Diamine mit Phosgen im stöchiometrischen Überschuß von Phosgen in mindestens einer Reaktionszone,
wobei man die Reaktionsbedingungen so wählt, daß zumindest die Reaktionskomponenten Diamin, Diisocyanat und Phosgen bei diesen Bedingungen gasförmig sind, und
wobei man der Reaktionszone mindestens einen diaminhaltigen und mindestens einen phosgenhaltigen Gasstrom zuführt,
wobei man aus dem erhaltenen, im wesentlichen gasförmigen Reaktionsgemisch überschüssiges Phosgen und entstandenes Chlorwasserstoffgas (HCl) abtrennt, man das abgetrennte, überschüssige Phosgen zumindest teilweise wieder in die Reaktion zurückführt,
und wobei man aus dem zurückgeführten Phosgenstrom Chlorwasserstoff so abtrennt, daß der Massenbruch von Chlorwasserstoff im phosgenhaltigen Strom vor der Vermischung mit dem aminhaltigen Strom mindestens 0,0001 Gew% und weniger als 15 Gew.-% beträgt.

2. Verfahren gemäß Anspruch 1, **dadurch gekennzeichnet, daß** dem diaminhaltigen und/oder phosgenhaltigen Gasstrom ein Inertmedium zusetzt, so daß das Gasvolumen von Inertmedium zu Amin und/oder zu Phosgen mehr als 0,0001 und bis zu 30 beträgt.

3. Verfahren gemäß einem der vorstehenden Ansprüche, **dadurch gekennzeichnet, daß** die Umsetzung von Phosgen mit Diamin im Reaktionsraum bei Absolutdrücken von mehr als 0,1 bar bis weniger als 20 bar erfolgt.

4. Verfahren gemäß einem der vorstehenden Ansprüche, **dadurch gekennzeichnet, daß** die Umsetzung von Phosgen mit Diamin im Reaktionsraum bei Temperaturen von mehr als 200 °C bis zu 600 °C erfolgt.

5. Verfahren gemäß einem der vorstehenden Ansprüche, **dadurch gekennzeichnet, daß** die mittlere Kontaktzeit des Umsetzungsgemisches zwischen 0,001 Sekunden und weniger als 5 Sekunden beträgt.

6. Verfahren gemäß einem der vorstehenden Ansprüche, **dadurch gekennzeichnet, daß** das molare Verhältnis von Phosgen zu Aminogruppen von 1,1 :1 bis 20 : 1 beträgt.

7. Verfahren gemäß einem der vorstehenden Ansprüche, **dadurch gekennzeichnet, daß** die Strömung im Reaktionsraum eine Bodensteinzahl von mehr als 10 aufweist.

8. Verfahren gemäß Anspruch 2, **dadurch gekennzeichnet, daß** das Inertmedium Chlorbenzol ist.

9. Verfahren gemäß einem der vorstehenden Ansprüche, **dadurch gekennzeichnet, daß** Phosgen und Amin im gasförmigen Zustand miteinander reagieren und Eduktströme und Zwischenprodukte im Verlauf der Reaktion während des Durchgangs durch den Reaktionsraum zu mindestens 95% in der Gasphase bleiben.

10. Verfahren gemäß einem der vorstehenden Ansprüche, **dadurch gekennzeichnet, daß** das Isocyanat ausgewählt ist aus der Gruppe bestehend aus 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemischen.

## Claims

1. A process for preparing diisocyanates by reacting the corresponding diamines with phosgene in a stoichiometric excess of phosgene in at least one reaction zone,
wherein the reaction conditions are selected so that at least the reaction components diamine,
diisocyanate and phosgene are gaseous under these conditions and
at least one diamine-comprising gas stream and at least one phosgene-comprising gas stream are fed into the reaction zone,
excess phosgene and hydrogen chloride gas (HCl) formed are separated off from the essentially gaseous reaction mixture obtained,
the excess phosgene which has been separated off is at least partly recirculated to the reaction,
and hydrogen chloride is separated off from the recirculated phosgene stream so that the mass fraction of hydrogen chloride in the phosgene-comprising stream prior to mixing with the amine-comprising stream is at least 0.0001% by weight and less than 15% by weight.

2. The process according to claim 1, wherein an inert medium is added to the diamine-comprising and/or phosgene-comprising gas stream so that the gas volume of inert medium to amine and/or to phosgene is greater than 0.0001 and up to 30.

3. The process according to either of the preceding claims, wherein the reaction of phosgene with diamine in the reaction space occurs at absolute pressures of from > 0.1 bar to < 20 bar.

4. The process according to any of the preceding claims, wherein the reaction of phosgene with diamine in the reaction space occurs at temperatures of from > 200°C to 600°C.

5. The process according to any of the preceding claims, wherein the mean contact time of the reaction mixture is in the range from 0.001 seconds to < 5 seconds.

6. The process according to any of the preceding claims, wherein the molar ratio of phosgene to amino groups is from 1.1 :1 to 20 : 1.

7. The process according to any of the preceding claims, wherein the flow in the reaction space has a Bodenstein number of more than 10.

8. The process according to claim 2, wherein the inert medium is chlorobenzene.

9. The process according to any of the preceding claims, wherein phosgene and amine react with one another in the gaseous state and feed streams and intermediates remain in the gas phase to an extent of at least 95% during the course of the reaction while passing through the reaction space.

10. The process according to any of the preceding claims, wherein the isocyanate is selected from the group consisting of 1,6-diisocyanatohexane, 1-isocyanato-3,3,5-trimethyl-5-(isocyanato-methyl) cyclohexane, 4,4'-di(isocyanatocyclohexyl)methane and tolylene diisocyanate isomer mixtures.

## Revendications

1. Procédé pour la préparation de diisocyanates par transformation des diamines correspondantes avec du phosgène, avec un excès stoechiométrique de phosgène dans au moins une zone de réaction,
en choisissant les conditions de réaction de manière telle qu'au moins les composants de réaction diamine,
diisocyanate et phosgène sont gazeux dans ces conditions, et
en alimentant dans la zone de réaction au moins un flux gazeux contenant une diamine et au moins un flux gazeux contenant du phosgène,
en séparant, du mélange réactionnel sensiblement gazeux obtenu, le phosgène en excès et le chlorure d'hydrogène (HCl) formé, en recyclant le phosgène en excès séparé au moins partiellement dans la réaction,
et en séparant le chlorure d'hydrogène du flux de phosgène recyclé de manière telle que la fraction massique du chlorure d'hydrogène dans le flux contenant du phosgène, avant le mélange avec le flux contenant l'amine, est d'au moins 0,0001% en poids et inférieure à 15% en poids.

2. Procédé selon la revendication 1, **caractérisé en ce qu'**on ajoute au flux gazeux contenant la diamine et/ou le phosgène un agent inerte de telle sorte que le volume gazeux d'agent inerte à amine et/ou à phosgène est supérieur à 0,0001 et jusqu'à 30.

3. Procédé selon l'une quelconque des revendications précédentes, **caractérisé en ce que** la transformation du phosgène avec la diamine dans l'espace de réaction a lieu à des pressions absolues supérieures à 0,1 bar jusqu'à moins de 20 bars.

4. Procédé selon l'une quelconque des revendications précédentes, **caractérisé en ce que** la transformation du phosgène avec la diamine dans l'espace de réaction a lieu à des températures de plus de 200°C jusqu'à 600°C.

5. Procédé selon l'une quelconque des revendications précédentes, **caractérisé en ce que** le temps de contact moyen du mélange de transformation vaut entre 0,001 seconde et moins de 5 secondes.

6. Procédé selon l'une quelconque des revendications précédentes, **caractérisé en ce que** le rapport molaire du phosgène aux groupes amino est de 1,1:1 à 20:1.

7. Procédé selon l'une quelconque des revendications précédentes, **caractérisé en ce que** le flux dans la chambre de réaction présente un nombre de Bodenstein supérieur à 10.

8. Procédé selon la revendication 2, **caractérisé en ce que** l'agent inerte est du chlorobenzène.

9. Procédé selon l'une quelconque des revendications précédentes, **caractérisé en ce que** le phosgène et l'amine réagissent l'un avec l'autre à l'état gazeux et les flux de produits de départ et les produits intermédiaires restent à raison d'au moins 95% dans la phase gazeuse au cours de la réaction, pendant le passage à travers l'espace de réaction.

10. Procédé selon l'une quelconque des revendications précédentes, **caractérisé en ce que** l'isocyanate est choisi dans le groupe constitué par le 1,6-diisocyanatohexane, le 1-isocyanato-3,3,5-triméthyl-5-(isocyanatométhyl)cyclohexane, le 4,4'-di(isocyanatocyclohexyl)méthane et les mélanges d'isomères de toluylènediisocyanate.
